# FASCICULE DE BREVET EUROPEEN

(11) **EP 1 722 829 B1**
(45) Date de publication et mention de la délivrance du brevet: **05.06.2013**
(21) Numéro de dépôt: 05739708.5
(22) Date de dépôt: 02.03.2005
(51) Int. Cl.: A61L 2/232, C09D 5/14, A01N 25/24

(54) **PROCEDE POUR REDUIRE LES PROPRIETES DE CONTAMINATION D'UNE SURFACE PAR DES MICROORGANISMES**
VERFAHREN ZUR REDUZIERUNG DER KONTAMINATIONSEIGENSCHAFTEN EINER OBERFLÄCHE DURCH MIKROORGANISMEN
METHOD FOR REDUCING THE CONTAMINATION PROPERTIES OF A SURFACE BY MICROORGANISMS

(30) Priorité: 03.03.2004 FR 0450438
(43) Date de publication de la demande: 22.11.2006
(73) Titulaire: Association pour les transferts de technologie Du Mans, 72000 Le Mans (FR); INSTITUT NATIONAL DE LA RECHERCHE AGRONOMIQUE (INRA), 75341 Paris Cedex 07 (FR)
(72) Inventeur: LEGEAY, Gilbert, F-72650 SAINT SATURNIN (FR); HONORE, Tanguy, F-72000 LE MANS (FR); BELLON-FONTAINE, Marie-Noëlle, F-91620 LA VILLE DU BOIS (FR); HERRY, Jean-Marie, F-91520 EGLY (FR)
(74) Mandataire: Catherine, Alain
(86) Numéro de dépôt international: PCT/FR2005/050142
(87) Numéro de publication internationale: WO 2005/084719

(56) Documents cités:
- EP-A- 0 194 770
- EP-A- 0 290 676
- FR-A- 2 342 074
- GB-A- 2 310 140
- US-A- 4 990 547
- US-A- 5 571 312

## Description

La présente invention se rapporte au domaine du traitement des surfaces contre le développement des microorganismes dans des environnements nécessitant des conditions rigoureuses d'asepsie, notamment dans le domaine des industries agroalimentaires, des industries de la santé, dans les lieux publics etc..

Le développement non contrôlé de microorganismes, en particulier des bactéries, des champignons ou encore des algues unicellulaires, mais aussi dans certains cas des virus, doit être le plus possible évité dans des environnements sensibles tels que les hôpitaux, les usines de production de médicaments, l'industrie agro-alimentaire, les cuisines collectives, ou encore les lieux publics.

La contamination des surfaces par des microorganismes a lieu sur toutes les surfaces en contact avec l'atmosphère, tels que les sols, le mobilier technique (table d'opération, lits d'hôpitaux, etc), les dispositifs de production utilisés dans l'industrie pharmaceutique et dans l'industrie agroalimentaire, notamment les réacteurs de fermentation.

En milieu hospitalier, la prévention à l'encontre de la contamination des locaux et du gros mobilier par des microorganismes revêt une importance considérable dans la lutte contre la propagation des maladies dites « nosocomiales ».

Dans l'industrie agro-alimentaire, ainsi que dans le domaine de la cuisine pour collectivité, la prévention de la contamination par des microorganismes de dispositifs de fabrication des aliments, notamment des plats préparés, ainsi que des dispositifs de cuisson des aliments, est essentielle pour éviter de provoquer des intoxications alimentaires, qui sont parfois mortelles pour le consommateur final.

De même, dans les industries mettant en oeuvre des procédés de fermentation en réacteur, il est nécessaire d'éviter la contamination des milieux de fermentation par des microorganismes exogènes non désirés.

Il est également important d'assurer une relative asepsie de nombreux lieux et objets d'usage publics, avec lesquels de nombreux individus sont en contact, afin d'éviter que des germes présents chez des individus affectés ne puissent facilement disséminer vers des individus sensibles, par exemple des individus immunodéprimés.

Aujourd'hui, l'asepsie des surfaces est réalisée, par exemple en milieu hospitalier, par application sur ces surfaces de solutions contenant des combinaisons d'agents antiseptiques et bactéricides. Toutefois, il est observé que certains des agents antiseptiques et bactéricides utilisés sont allergisants et présentent donc des inconvénients vis-à-vis de la santé humaine.

Par ailleurs, certains de ces agents, pourtant efficaces, ne peuvent être utilisés dans l'industrie agroalimentaire ou dans des locaux culinaires en raison de leur toxicité lors de leur ingestion par le consommateur final.

Il existe donc un besoin dans l'état de la technique pour des procédés et des compositions de traitement de surfaces ayant une aire importante, et qui peut atteindre dans certains cas plus de 1000 m², afin d'empêcher, ou à tout le moins de réduire, la contamination de ces surfaces par des microorganismes, et tout particulièrement des microorganismes pathogène pour l'homme ou l'animal.

Les demandes de brevet FR 2342074, EP 0194770 et US 49990547 traitent du problème relatif à la contamination des surfaces par des agents nuisibles, comme les microorganismes. La solution apportée par ces documents de l'art antérieur consiste à inhiber la formation d'un biofilm en utilisant une couche polymérique libérant un agent bactéricide, germicide on antifongique. La demande FR 2342074 propose de former à la surface des matériaux une pellicule obtenue par projection d'une composition comprenant notamment un désinfectant offrant des propriétés biocides. La demande EP 0194770 décrit une composition formant un film polymérique adhérent permettant la libération prolongée d'un agent anti-microbien qu'elle renferme. La demande US 49990547 propose enfin de recouvrir la surface d'objets avec une composition comprenant un polymère encapsulant un composé algicide de type dialkyldiméthyl ammonium, ladite composition permettant la libération prolongée du composé algicide. Une telle approche consiste à former une barrière chimique à la surface des matériaux. Le procédé selon la présente invention diffère profondément des procédés tels que rapportés ci-dessus.

Compte-tenu de l'aire importante des surfaces à traiter, il est essentiel que ces procédés anti-biocontamination soient simples à réaliser et peu coûteux.

Un procédé et une composition pour empêcher ou réduire la biocontamination de larges surfaces sont fournis par la présente invention.

Le procédé selon l'invention est défini dans la revendication 1, à savoir un procédé pour réduire les propriétés de la contamination par des micro-organismes d'une surface. d'un matériau minéral, ladite surface avant une aire d'au moins 0,1 m², ledit procédé comprenant les étapes suivantes :
a) application, sur la surface à traiter, d'une couche d'une solution ou d'une suspension aqueuse d'un matériau polymère hydrophile, tel que ledit matériau polymère hydrophile adhère sur ladite surface à traiter par création de liaisons non-covalentes entre le matériau minéral de la surface traitée et la couche du matériau polymère, comprenant un agent durcisseur consistant en des particules de silices dispersées dans le milieu aqueux, ladite suspension aqueuse ayant une teneur en particules de silice allant de 50 g.l⁻¹ à 250 g.l⁻¹ ;
b) séchage de la surface traitée à l'étape a), pour obtenir ladite surface recouverte par une couche dudit matériau polymère hydrophile, ladite couche avant une dureté de surface supérieure à 100 N.mm⁻² déterminée par une méthode de micro-indentation, telle qu'un indentateur de forme pyramidale et d'angle au sommet de 136° piloté par un microscope pénètre dans le matériau sous une charge de 25 mN en 20 secondes à une température de 20 °C et un taux d'humidité de 45 %, l'indentateur ne pénétrant pas à plus du quart de !'épaisseur de la couche étudiée.
La composition selon l'invention est définie dans la revendication 9, à savoir une composition pour le traitement d'une surface avant une aire d'au moins 1 m2 à, l'encontre d'une contamination par des micro-organismes, caractérisée en ce que ladite composition consiste en une solution aqueuse d'un matériau polymère hydrophile, ladite composition comprenant des particules de silice fumée à la teneur finale allant de 50g.l⁻¹ à 250g.l⁻¹, et comprenant au moins un agent bactéricide ou un agent conservateur.

De manière surprenante, il est montré selon l'invention qu'une couche de polymère hydrophile, qui empêche l'adhésion et le développement des cellules de microorganismes, peut être appliquée avec succès sur une large surface à traiter, d'au moins 0,1 m², et allant jusqu'à plus de 1000 m², en l'absence de création de liaisons covalentes entre la couche de polymère hydrophile et la surface traitée. On a ainsi montré que la couche de polymère hydrophile reste en place intacte sur la surface traitée pendant une durée de plusieurs heures, voire plusieurs jours, selon les contraintes mécaniques qui sont subies par ladite surface traitée.

A partir de ce résultat surprenant, le demandeur a mis au point un procédé pour réduire les propriétés de contamination par des microorganismes d'une surface d'un matériau minéral, par exemple de verre, de céramique, de porcelaine, de ciment, de béton ou de métal, ledit procédé comprenant une étape au cours de laquelle on applique sur la surface à traiter une couche d'une solution d'une suspension aqueuse d'un matériau polymère hydrophile, sans créer de liaisons covalentes entre la couche de polymère hydrophile et la surface sur laquelle cette couche de polymère est appliquée.

L'invention a pour objet un procédé pour réduire les propriétés de contamination par des micro-organismes d'une surface d'un matériau minéral, ladite surface ayant une aire d'au moins 0,1 m², ledit procédé comprenant les étapes suivantes :
a) application, sur la surface à traiter, d'une couche d'une solution ou d'une suspension aqueuse d'un matériau polymère hydrophile ;
b) séchage de la surface traitée à l'étape a), pour obtenir ladite surface recouverte par une couche dudit matériau polymère hydrophile.

La couche du matériau polymère hydrophile appliquée grâce au procédé ci-dessus adhère sur la surface traitée par la simple création de liaisons faibles non-covalentes entre le matériau minéral de la surface traitée et la couche du matériau polymère, en particulier des liaisons faibles électrostatiques, des liaisons hydrogènes ou encore des forces de Van der Waals. L'adhérence de la couche du matériau polymère hydrophile sur la surface du matériau minéral traitée n'implique donc aucune réaction chimique entre ce matériau polymère et le matériau minéral. Ainsi, les surfaces traitées par le procédé de l'invention ne sont pas modifiées chimiquement. Ces surfaces peuvent donc être traitées par le procédé de l'invention de manière répétée sans subir une quelconque modification ou altération.

Néanmoins, de manière surprenante, la couche de matériau polymère hydrophile adhère de manière stable sur la surface du matériau minéral. Sans vouloir être lié par une quelconque théorie, le demandeur pense que les interactions faibles (liaisons hydrogène, forces électrostatiques, forces de Van der Waals) entre la surface traitée et la couche de polymère hydrophile suffisent à elles seules à provoquer l'adhérence de ladite couche polymère. Les propriétés d'adhérence de la couche polymère sur la surface traitée sont suffisantes pour que la couche polymère reste en place et recouvre efficacement et complètement la surface traitée, jusqu'au moment du traitement suivant de cette surface par le procédé de l'invention, par exemple 7, 6, 5, 4, 3, 2 ou 1 jour(s) après le traitement antérieur.

Grâce au procédé de l'invention, on inhibe ou on bloque la bioadhésion des microorganismes sur le matériau minéral, bioadhésion qui a lieu par établissement de forces électrostatiques entre les cellules bactériennes ou fongiques et le matériau minéral. Selon le procédé de l'invention, la bioadhésion des microorganismes sur une surface en inhibant ou en bloquant l'accrochage mécanique de ces microorganismes sur la surface, souvent poreuse ou rugueuse, du matériau minéral.

Grâce au procédé de l'invention, on empêche également la formation de biofilms constitués par un tapis de cellules bactériennes ou fongiques plus ou moins épais, qui se forment rapidement au cours du temps, surtout dans des environnements en atmosphère humide.

Au contraire, du fait de la présence de la couche du matériau polymère hydrophile, une surface d'un matériau minéral traité selon le procédé ci-dessus possède une faible énergie de surface, ce qui empêche l'adhésion des microorganismes, notamment les cellules bactériennes ou fongiques.

De plus, la surface externe, en contact avec l'environnement extérieur, de la couche du matériau polymère hydrophile est sensiblement plane, non poreuse et non rugueuse, ce qui réduit encore la possibilité d'accrochage mécanique des microorganismes, notamment des cellules bactériennes ou fongiques, sur la surface traitée et réduit par conséquent le phénomène de biocontamination de la surface traitée.

Par « matériau minéral », on entend selon l'invention un matériau constitué de verre, de céramique, de porcelaine, de ciment, de béton ou encore un matériau métallique.

Selon un premier mode de réalisation préféré, le matériau minéral est du verre organique ou du verre minéral.

Selon un second mode de réalisation préféré, le matériau minéral est un matériau métallique, tel que l'acier, par exemple un acier inoxydable, ou encore l'aluminium.

Comme cela a déjà été mentionné précédemment, la surface du matériau minéral traitée selon le procédé de l'invention consiste en une surface large ayant une aire d'au moins 0,1 m². L'aire totale de la surface à traiter n'est techniquement limitée que par la capacité du dispositif utilisé pour l'application de la solution ou de la suspension aqueuse du matériau polymère hydrophile. Par exemple, pour traiter des aires supérieures à 100 par le procédé selon l'invention, on peut utiliser des engins de nettoyage qui sont couramment utilisés pour le nettoyage des sols d'usine ou des lieux publics.

Ainsi, de manière surprenante, l'aire minimale de la surface qui peut être traitée par le procédé de l'invention est d'au moins 0,1 m² et peut être d'au moins 1 m², 2 m², 3 m², 4 m², 5 m², 10 m², 50 m² ou d'au moins 100 m². Des surfaces allant jusqu'à 1000 m² peuvent être traitées.

Pour mettre en oeuvre le procédé de l'invention pour le traitement de surfaces ayant une aire inférieure à 100 m², on peut utiliser des dispositifs adaptés tels que des balais couramment utilisés pour le nettoyage de petites aires.

Selon un premier aspect, la surface du matériau minéral à traiter consiste en la surface d'un sol, notamment d'un sol d'une pièce d'hôpital, par exemple le sol d'une chambre d'hôpital, le sol d'une pièce où sont pratiqués des soins aux patients, y compris le sol d'une salle d'un bloc opératoire. Il peut également s'agir d'un sol d'une usine de fabrication de produits agro-alimentaires, comme des produits agro-alimentaires laitiers (lait, fromage, crème glacée, etc.), ou encore le sol d'une cuisine pour collectivité.

La surface du matériau minéral à traiter peut également consister en la surface de dispositifs ou de mobiliers utilisés dans les hôpitaux ou dans l'industrie, telle que la surface du plateau d'une table d'hôpital, y compris une table d'opération, ou encore un plan de travail culinaire.

La surface du matériau minéral à traiter peut également consister en la surface de tubulures ou tuyaux de circulation de fluides, notamment dans les hôpitaux et dans l'industrie agro-alimentaire, y compris les tubulures ou des tuyaux de circulation de gaz ou de fluides liquides tels que l'eau.

Selon encore un autre aspect, la surface du matériau minéral à traiter peut consister en la surface interne et/ou externe d'un réacteur de fermentation utilisé pour la fabrication de divers métabolites, par exemple dans l'industrie agro-alimentaire ou encore dans l'industrie pharmaceutique.

De préférence, à l'étape a) du procédé, la solution ou la suspension aqueuse comprend le matériau polymère hydrophile à une concentration comprise entre 0,5 % et 5%, mieux entre 1% et 3%, par rapport au poids total de la solution ou de la suspension aqueuse.

Plus la concentration du matériau polymère hydrophile en solution aqueuse est élevée, plus la viscosité de la solution aqueuse est grande, et plus l'épaisseur de la couche du matériau polymère hydrophile sur la surface traitée est épaisse et protectrice. Selon les contraintes mécaniques qu'est susceptible de subir la surface à traiter, comme par exemple le déplacement fréquent d'objets sur roulettes, le passage de nombreux individus, etc., on adapte la concentration du matériau polymère hydrophile de telle manière à ce que la couche du matériau polymère hydrophile ait une épaisseur suffisante pour empêcher le développement des microorganismes jusqu'au traitement suivant de ladite surface par le procédé.

Afin d'obtenir une couche suffisamment protectrice de polymère hydrophile, c'est-à-dire une couche de polymère hydrophile suffisamment épaisse pour couvrir sans difficulté excessive l'ensemble de la surface à traiter, on adapte la quantité du matériau polymère hydrophile par rapport au poids total de la solution, de manière à obtenir une solution aqueuse du matériau polymère hydrophile ayant une viscosité d'au moins 1 centipoise.

La viscosité de la solution ou suspension aqueuse du matériau polymère hydrophile peut aller jusqu'à 10 centipoises, lorsque l'on souhaite appliquer une couche de matériau polymère hydrophile de grande épaisseur, par exemple sur des surfaces de matériaux minéral destinés à subir de nombreuses contraintes mécaniques.

Selon l'invention, les mesures de viscosité sont réalisées dans un système Brookfield selon une technique normalisée, à température contrôlée (25°C), en utilisant un viscosimètre DIN équipé d'une aiguille de type DIN 30D, et pour une vitesse de rotation de 300 à 500 tours/minute. L'équipement donne automatiquement la valeur de viscosité qui est une fonction de couple de rotation.

De manière tout à fait préférée, le matériau polymère hydrophile consiste en un polymère ou une combinaison de polymères choisie parmi les celluloses et leurs dérivés, les polyacrylamides et leurs copolymères, la polyvinylpyrrolidone (PVP) et ses copolymères, les copolymères d'acétate de vinyle et d'alcool vinylique, les polyéthylène glycols, les polypropylène glycols, les polyacrylates hydrophiles, les polyméthacrylates hydrophiles, les polyosides et les chitosans.

De préférence, le matériau polymère hydrophile est choisi parmi les polymères hydrophiles suivants :
- les celluloses et leurs dérivés, telles que l'hydroxypropylméthylcellulose (HPMC), par exemple la HPMC E4M commercialisée par la Société DOW CHEMICALS, ou celle dénommée Aqualon commercialisée par la Société Herculès, ou encore la carboxyméthylcellulose (CMC) commercialisée par la Société DOW CHEMICALS:
- les polyacrylamides et leurs copolymères, tels que ceux commercialisés par la Société SIGMA (UPSALA, Suède) ;
- la polyvinylpyrrolidone (PVP) et ses copolymères, tels que ceux commercialisés par la Société BASF/LASERSON, comme la famille des Kollidon ;
- les copolymères de l'acétate de vinyle, tels que les copolymères de polyacétate de vinyle et d'alcool polyvinylique commercialisés sous le nom de Mowiol par la Société HOECHST/CLARIANT.
- les polyéthylènes glycols, tels que ceux commercialisés par la Société SIGMA.
- les polypropylènes glycols ;
- les poly (méth)acrylates hydrophiles, tels que ceux commercialisés par les Sociétés DEGALAN ou DEGUSSA ;
- les polyosides ;
- les chitosans, tels que ceux commercialisés par la Société SIGMA.

On entend par matériau polymère hydrophile selon l'invention, aussi bien un matériau polymère constitué de l'un des polymères hydrophiles tels que définis ci-dessus qu'un mélange de plusieurs des polymères hydrophiles ci-dessus, en général un mélange de deux ou trois des polymères hydrophiles ci-dessus.

On a montré selon l'invention que des surfaces de verre ou d'acier recouvertes d'une couche de matériaux polymères hydrophiles avec le procédé de l'invention était stable sur la surface traitée pendant une durée allant jusqu'à 96 heures.

On a aussi montré qu'une surface de verre ou d'acier traité, selon le procédé de l'invention, par une couche d'un matériau polymère hydrophile, après immersion pendant 72 heures dans une solution contenant des cellules bactériennes, telles que *Escherichia coli* et *Staphyloccus epidermis* à des concentrations comprises entre 10⁶ et 3.10⁶ par gramme de solution, n'était toujours pas colonisée par ces bactéries. Il est constaté une mortalité significative avec les cellules de *Escherichia coli,* avec une diminution d'un facteur 2 par rapport au nombre de cellules inoculées au départ. Pour *Staphylococus epidermis* la mortalité après 72 heures est presque totale. Notamment, il n'est observé aucun film de cellules bactériennes à la surface du matériau traité. En comparaison, le même support, non traité selon le procédé de l'invention, possède, au bout de 72 heures d'immersion dans la même solution contenant des bactéries, un taux de recouvrement par les bactéries de l'ordre de 70% de son aire totale.

Avec une surface traitée selon le procédé de l'invention, il est observé un glissement des cellules bactériennes sur la couche de polymère hydrophile, sans fixation de ces cellules à la surface de ladite couche de polymère hydrophile.

A l'étape a) du procédé selon l'invention, l'application du matériau polymère hydrophile est réalisée, selon le type et la géométrie du dispositif à traiter, soit par trempage de la surface à traiter, soit par application du matériau en polymère hydrophile sur la surface à traiter à l'aid e d'une brosse, d'un pinceau, ou de tout type de dispositif de pulvérisation, tel qu'un pistolet de pulvérisation de peinture ou encore un dispositif de nettoyeur à haute pression, tel qu'un dispositif de type Karcher®.

On peut aussi utiliser une surface de toile, par exemple une serpillière, préalablement imprégnée de la solution ou suspension du matériau polymère hydrophile

A l'étape b) du procédé, le séchage peut être réalisé simplement sans intervention humaine, pendant une durée suffisante nécessaire à l'évaporation du solvant aqueux en contact avec l'atmosphère environnante.

L'étape b) de séchage peut être également réalisée par l'application, sur la surface traitée selon l'étape a), d'un courant d'air à température ambiante d'environ 20 à 25°C, ou encore d'un courant d'air chaud à une température pouvant aller jusqu'à 55°C, pendant une durée suffisante pour provoquer l'évaporation du solvant aqueux, par exemple environ 10 minutes, sur chaque partie de la surface traitée.

Il est également décrit une composition pour le traitement d'une surface d'un matériau minéral ayant une aire d'au moins 0,1 m² à l'encontre d'une contamination par des microorganismes selon la revendication 9.

Selon la viscosité de la solution ou de la suspension aqueuse du matériau polymère hydrophile, on a observé que le procédé de l'invention permettait d'appliquer sur la surface à traiter une couche de polymère hydrophile allant de 100 nanomètres à 10 µm environ.

A titre d'exemple, une valeur de viscosité de la solution ou suspension du matériau polymère hydrophile de l'ordre de 5 à 10 centipoises (cPs) est obtenue pour une concentration de 1 % en poids de PVP (Kollidon K90 commercialisée par la Société BASF) ou pour une concentration de 0,2% en poids de HPMC (E4M commercialisée par la Société DOW CHEMICALS).

Par « solution ou suspension aqueuse » selon l'invention, on entend principalement que le matériau polymère hydrophile utilisé est en solution ou en suspension dans de l'eau, éventuellement en présence d'une faible proportion, par exemple de 0,1% à 5% (V :V), d'un solvant miscible dans l'eau tel que l'éthanol ou une cétone, y compris l'acétone, ou tout autre diluant autorisé par les réglementations administratives.

La solution aqueuse contenant le matériau polymère hydrophile peut également contenir un ou plusieurs additifs destinés à améliorer son étalement sur la surface à traiter tels que des agents tensioactifs neutres ou à caractère acide ou basique. On préfère toutefois des additifs tensio-actifs neutres tels que les polyéthylènes et les polypropylènes glycols, le polyoxyéthyle sorbitol stéarate (Tween®), un polyéther ou encore un polysitoxane-polyéther. Ces produits sont notamment commercialisés par la Société BYK CHEMIE.

La solution aqueuse du matériau polymère hydrophile peut également comprendre un agent promoteur d'adhérence afin d'améliorer le maintien de la couche de polymère sur la surface traitée, tels que des organosiliclés, des phosphoaluminates ou des zircoaluminates, ou des phosphates. Ces derniers sont les plus connus. Ils sont tous d'origine commerciale. Dans certains cas, le caractère acide ou basique est à prendre en compte en fonction de la nature chimique du support sur lequel est déposé le revêtement, afin d'éviter une altération de la surface à traiter.

De manière avantageuse, la solution aqueuse du matériau polymère hydrophile contient un ou plusieurs agents antiseptiques, un ou plusieurs agents anti-bactériens ou un ou plusieurs agents conservateurs, ce qui permet de réduire encore la prolifération des cellules bactériennes ou fongiques. On peut par exemple utiliser, comme agent conservateur, le benzoate de sodium, de préférence à la concentration pondérale finale de 1%, ou encore l'azide de sodium, de préférence à la concentration pondérale finale de 1 %.

Pour traiter, grâce au procédé de l'invention, des surfaces destinées à subir de fortes ou nombreuses contraintes mécaniques, *on ajoute* à la solution aqueuse du matériau polymère hydrophile un agent de durcissement capable d'améliorer la résistance de la couche à l'abrasion.

De manière tout à fait préférée, l'agent de durcissement consiste en des particules de silice synthétique en dispersion en milieu liquide aqueux. Les particules de silice synthétique sont constituées de polymères d'acide silicique dont la structure de masse est produite par réticulation des tétraèdres de SiO₄. A la surface des particules, la structure des polymères se termine par des groupes siloxane et des groupes silanol. On utilise de préférence des particules de silice fumée (« fumed silica ») hydrophile. Les silices fumées sont préparées par hydrolyse de tétrachlorure de silicium (TiCl₄) en phase vapeur dans une flamme d'hydrogène et d'oxygène à une température d'au moins 1000°C ayant une surface spécifique comprise entre 200 et 400 m²/g, mieux entre 250 et 400 m²/g, et de manière tout à fait préférée entre 300 et 400 m²/g, par exemple des silices ayant une surface spécifique de 315 m²/g ou de 342 m²/g. On peut utiliser notamment des dispersions de silice commercialisées sous la dénomination Aérosil par la Société DEGUSSA. Parmi les dispersions de silice de type Aérosil, on préfère utiliser celles ayant les grades K315 et K 342, respectivement.

De manière tout à fait préférée, on utilise des particules de silice dispersées en milieu liquide aqueux ayant un caractère légèrement acide, comme les silices K315 et K342 ci-dessus.

En revanche, on évite le recours aux dispersions de particules de silice fortement basiques, parce que il y a un risque de dissolution de cette silice dans le milieu pour former des silicates, ce qui n'est pas souhaitable.

De préférence, selon cet aspect particulier du procédé, la solution ou dispersion aqueuse du matériau polymère contient de 50 g/l à 250 g/l de particules de silice, et de manière tout à fait préférée de 70 g/l à 200 g/l de particules de silice.

On a montré selon l'invention que la couche de polymère hydrophile obtenue par application du procédé avec une solution aqueuse de HPMC de type E4M à 0,2% en poids dans l'eau avait une dureté de 2,33 N.mm⁻², comme déterminé par microindentation (système Fisher H100C). Un indenteur de forme pyramidale (136°) piloté par un microscope pénètre dans le matériau sous une charge de 25mN en 20sec (température 22°C et taux d'humidité 45%) de façon à ce que l'indenteur ne pénètre pas à plus du quart de l'épaisseur de la couche étudiée. La mesure de la profondeur de pénétration atteinte à charge maximale permet de calculer la dureté.

On a aussi montré selon l'invention que la dureté de la couche polymère obtenue par application du procédé avec une solution aqueuse de HPMC E4M à 0,2% dans l'eau contenant 50% en poids d'une dispersion de particules de silice de type K315 à une dureté d'environ 350 N.mm⁻², comme déterminé par microindentation.

La composition selon l'invention comprend de préférence de 0,5% à 5% en poids, par rapport au poids total de la solution aqueuse, dudit matériau polymère hydrophile.

*On décrit également* une surface d'un matériau minéral ayant une aire d'au moins 1 m² et résistante aux contaminations par des micro-organismes, caractérisée en ce que ladite surface de verre, de céramique ou d'acier est recouverte par une couche d'un matériau polymère hydrophile.

La surface ci-dessus est caractérisée en ce que la couche de matériau polymère hydrophile comprend les particules de silice et possède une dureté de surface supérieure à 100 N.mm ⁻². Les résultats avec des *Eschericcia coli* et des *Staphylococcus epidermis* montrent une absence d'adhésion après 24 heures

L'invention est en outre illustrée par les exemples suivants.

### EXEMPLES :

### EXEMPLE 1 : Essai d'adhérence et de biocontamination sur une surface de verre traitée avec un polymère hydrophile selon le procédé de l'invention

### A. Protocole de préparation de plaques de verre recouvertes par un polymère hydrophile.

Les plaques de verre sont lavées (i) soit avec de l'eau savonneuse puis rincées à l'eau pure, (ii) soit avec un solvant organique propre (et notamment alcool éthylique). Dans tous les cas les plaques sont séchées à l'air ambiant si possible sous un courant d'air chaud, pendant quelques minutes. Ces plaques sont conservées en milieu propre.

Puis, la surface des plaques est recouverte d'une couche de polymère hydrophile par immersion dans un bain d'une solution de HPMC à 0,2% (poids/poids) pendant une durée de 72 heures

### B. Résultats des essais de bioadhésion.

Des tests de bioadhésion sont effectués en milieu physiologique soit en :
- système statique, les objets sont mis à tremper plusieurs jours et des prélèvements sont effectués régulièrement,
- soit en système dynamique, par immersion de la surface traitée selon le procédé de l'invention dans une cuve contenant une solution de milieu contenant des microorganisme. Des courants du milieu liquide sont créés dans la cuve, afin (i) de tester la capacité d'adhérence de la couche de polymère hydrophile sur la surface du support et (ii) de tester la capacité anti-biocontamination de la surface traitée. Des solutions présentant une concentration de l'ordre de 1.10⁶ microorganismes cm⁻³ sont utilisées.

### Résultats obtenus par application du procédé de l'invention sur une surface de verre :

Des plaquettes de verre recouvertes d'un polymère hydrophile (HPMC ou hydroxypropyl méthylcellulose) sont mises en contact pendant des durées allant jusqu'à 72 heures. Dans certains cas les plaques sont lavées avec un détergent alcalin phosphaté (RBS).

Les résultats sont représentés dans le Tableau 1 ci-dessous.

**Tableau 1**

| **Surface** | **AVANT TEST** | | **APRES TEST** |
|---|---|---|---|
| | Traité HPMC | + préparation | % de surface recouverte par les microorganismes |
| **Verre** | non | Lavage RBS | 70% |
| | oui | pas de lavage | 0% |
| | oui | lavage RBS | 0% < S < 10% |
| **Acier** | non | Pas de lavage | 100% |
| | oui | Lavage RBS | 10% |

### EXEMPLE 2: Essai d'adhérence et de biocontamination sur une surface de verre traitée avec une association d'un polymère hydrophile avec des particules de silice selon le procédé de l'invention.

### A. Protocole de préparation des plaques de verre recouvertes par une couche contenant une charge minérale de particules de silice.

Préparation des plaques de verre : celles-ci sont lavées soit avec de l'eau savonneuse puis rincées à l'eau pure, soit avec un solvant organique propre (et notamment alcool éthylique). Dans tous les cas les plaques sont séchées à l'air ambiant si possible sous un courant d'air chaud, pendant quelques minutes. Ces plaques sont conservées en milieu propre.

Puis, la surface des plaques est recouverte d'une couche d'un polymère hydrophile :
- soit par immersion dans un bain d'une solution de HPMC à 0,2% (poids/poids) pendant une durée de 72 heures ;
- soit par immersion dans un bain d'une solution de 100 ml de HPMC à 0,2% (poids/poids) à laquelle on a ajouté 1,33 ml d'une solution aqueuse de particules de silice K315 à 15.0 g/l.

### B. Résultats des essais de bioadhésion.

Des plaquettes de verre recouvertes d'un polymère hydrophile (HPMC ou hydroxypropyl méthylcellulose) et contenant une charge minérale de particules de silice sont mises en contact pendant des durées allant jusqu'à 72 heures. Dans certains cas les plaques sont lavées avec un détergent alcalin phosphaté (RBS). La composition de la solution de polymère hydrophile est constituée de 100 ml d'une solution aqueuse de HPMC à 0,2% (poids/poids) à laquelle on ajoute 1,33 ml d'une solution aqueuse de particules de silice K315 à 150 g.l⁻¹, ce qui correspond, pour l'extrait sec, à 50 parties en poids de polymère et 50 parties en poids de particules de silice.

Les résultats sont représentés sur le Tableau 2 ci-dessous.

**Tableau 2:**

| **Souche** | **Sans dépôt** | **Dépôt HPMC + Silice + rinçage à l'eau distillée** | **Dépôt HPMC + Silice + nettoyage RBS35 à 2%** |
|---|---|---|---|
| ***E. coli*** | Adhésion des microorganismes au bout de 24 heures % de surface recouverte par les microorganismes : 70% | Pas d'adhésion des microorganismes au bout de 24h | les taches que l'on voyait précédemment ont disparu (surface plus uniforme) Faible adhésion des microorganismes au bout de 24h |
| | | % de surface recouverte par les microorganismes: 0% | |
| | | | % de surface recouverte par les microorganismes: 2 % |
| ***S***. ***epidermis*** | | Avant test : surface très opaque (difficile de faire la mise au point) | Avant test : les taches que l'on voyait précédemment ont disparu (surface plus uniforme) |
| | | A priori, pas d'adhésion des microorganismes au bout de 24h, mais la surface s'en va par petits morceaux. | Forte adhésion des microorganismes au bout de 24h |
| | | % de surface recouverte par les microorganismes: 0 % | % de surface recouverte par les microorganismes: 50% |

## Revendications

1. Procédé pour réduire les propriétés de contamination par des micro-organismes d'une surface d'un matériau minéral, ladite surface ayant une aire d'au moins 0,1 m², ledit procédé comprenant les étapes suivantes :
a) application, sur la surface à traiter, d'une couche d'une solution ou d'une suspension aqueuse d'un matériau polymère hydrophile, tel que ledit matériau polymère hydrophile adhère sur ladite surface à traiter par création de liaisons non-covalentes entre le matériau minéral de la surface traitée et la couche du matériau polymère, comprenant un agent durcisseur consistant en des particules de silices dispersées dans le milieu aqueux, ladite suspension aqueuse ayant une teneur en particules de silice allant de 50 g.l⁻¹ à 250 g.l⁻¹;
b) séchage de la surface traitée à l'étape a), pour obtenir ladite surface recouverte par une couche dudit matériau polymère hydrophile, ladite couche ayant une dureté de surface supérieure à 100 N.mm⁻², déterminée par une méthode de micro-indentation, telle qu'un indentateur de forme pyramidale et d'angle au sommet de 136° piloté par un microscope pénètre dans le matériau sous une charge de 25 mN en 20 secondes à une température de 20 °C et un taux d'humidité de 45 %, l'indentateur ne pénétrant pas à plus du quart de l'épaisseur de la couche étudiée..

2. Procédé selon la revendication 1, **caractérisé en ce que** le matériau minéral est du verre minéral.

3. Procédé selon la revendication 1, **caractérisé en ce que** le matériau minéral est choisi parmi la céramique, la porcelaine, le ciment ou le béton.

4. Procédé selon la revendication 1, **caractérisé en ce que** le matériau minéral est un matériau métallique, tel que l'acier ou l'aluminium.

5. Procédé selon l'une des revendications 1 à 4, **caractérisé en ce que** la surface de matériau minéral consiste en la surface d'un sol, d'un plan de travail culinaire, d'une table, d'un lit, d'un réacteur, ou encore d'une tubulure.

6. Procédé selon l'une des revendications 1 à 5, **caractérisé en ce que**, à l'étape a), la solution ou la suspension aqueuse comprend le matériau polymère hydrophile à une concentration comprise entre 0,5% et 5% en poids, préférentiellement entre 1% et 3% en poids, par rapport au poids total de la solution ou de la suspension aqueuse.

7. Procédé selon la revendication 6, **caractérisé en ce que** le matériau polymère hydrophile est choisi parmi les celluloses et leurs dérivés, les polyacrylamides et leurs copolymères, la polyvinylpyrrolidone (PVP) et ses copolymères, les copolymères d'acétate de vinyle et d'alcool vinylique, les polyéthylène glycols, les polypropylène glycols, les polyacrylates hydrophiles, les polyméthacrylates hydrophiles, les polyosides et les chitosans.

8. Procédé selon l'une des revendications 1 à 6, **caractérisé en ce qu'**à l'étape a), l'application du matériau polymère hydrophile est réalisé par trempage de la surface à traiter, ou par application du matériau sur la surface à l'aide d'un pinceau, d'un rouleau ou d'un dispositif de pulvérisation.

9. Composition pour le traitement d'une surface ayant une aire d'au moins 1 m² à l'encontre d'une contamination par des micro-organismes apte à la mise en oeuvre du procédé selon une des revendications 1 à 8, ladite composition consistant en une solution aqueuse d'un matériau polymère hydrophile, ladite composition comprenant des particules de silice fumée à la teneur finale allant de 50g.l⁻¹ à 250g.l⁻¹, et comprenant au moins un agent bactéricide ou un agent conservateur.

## Patentansprüche

1. Verfahren zum Reduzieren der Kontaminierbarkeit einer Oberfläche eines mineralischen Materials durch Mikroorganismen, wobei die Oberfläche einen Flächeninhalt von wenigstens 0,1 m² aufweist, wobei das Verfahren die folgenden Schritte umfasst:
a) Auftragen einer Schicht aus einer wässrigen Lösung oder Suspension eines hydrophilen polymeren Materials auf die zu behandelnde Oberfläche in einer solchen Weise, dass das hydrophile polymere Material auf der zu behandelnden Oberfläche haftet, durch Schaffung von nichtkovalenten Bindungen zwischen dem mineralischen Material der behandelten Oberfläche und der Schicht aus dem polymeren Material, das ein Härtungsmittel umfasst, welches aus Siliciumoxidteilchen besteht, die in dem wässrigen Medium dispergiert sind, wobei die wässrige Suspension einen Gehalt an Siliciumoxidteilchen von 50 g/l bis 250 g/l aufweist;
b) Trocknen der in Schritt a) behandelten Oberfläche, so dass die Oberfläche von einer Schicht des hydrophilen polymeren Materials bedeckt ist, wobei die Schicht eine Oberflächenhärte von über 100 N/mm² aufweist, bestimmt nach einem solchen Mikroeindruck-Härteprüfverfahren, dass ein Prüfstempel in Pyramidenform mit einem Öffnungswinkel von 136°, der durch ein Mikroskop gesteuert wird, unter einer Last von 25 mN in 20 Sekunden bei einer Temperatur von 20 °C und einer Luftfeuchtigkeit von 45% in das Material eindringt, wobei der Prüfstempel nicht weiter als bis zu einem Viertel der Dicke der untersuchten Schicht eindringt.

2. Verfahren gemäß Anspruch 1, **dadurch gekennzeichnet, dass** das mineralische Material ein Mineralglas ist.

3. Verfahren gemäß Anspruch 1, **dadurch gekennzeichnet, dass** das mineralische Material aus Keramik, Porzellan, Zement oder Beton ausgewählt ist.

4. Verfahren gemäß Anspruch 1, **dadurch gekennzeichnet, dass** das mineralische Material ein metallisches Material, wie Stahl oder Aluminium, ist.

5. Verfahren gemäß einem der Ansprüche 1 bis 4, **dadurch gekennzeichnet, dass** die Oberfläche des mineralischen Materials aus der Oberfläche eines Bodens, einer Küchenarbeitsfläche, einem Tisch, einem Bett, einem Reaktor oder auch einer Rohrleitung besteht.

6. Verfahren gemäß einem der Ansprüche 1 bis 5, **dadurch gekennzeichnet, dass** im Schritt a) die wässrige Lösung oder Suspension das hydrophile polymere Material in einer Konzentration im Bereich von 0,5 bis 5 Gew.-%, vorzugsweise 1 bis 3 Gew.-%, umfasst, bezogen auf das Gesamtgewicht der wässrigen Lösung oder Suspension.

7. Verfahren gemäß Anspruch 6, **dadurch gekennzeichnet, dass** das hydrophile polymere Material aus Cellulosen und ihren Derivaten, Polyacrylamiden und ihren Copolymeren, Polyvinylpyrrolidon (PVP) und seinen Copolymeren, Vinylacetat-Vinylalkohol-Copolymeren, Polyethylenglycolen, Polypropylenglycolen, hydrophilen Polyacrylaten, hydrophilen Polymethacrylaten, Polysacchariden und Chitosanen ausgewählt ist.

8. Verfahren gemäß einem der Ansprüche 1 bis 6, **dadurch gekennzeichnet, dass** im Schritt a) die Auftragung des hydrophilen polymeren Materials durch Eintauchen der zu behandelnden Oberfläche oder durch Auftragung des Materials auf die Oberfläche mittels eines Pinsels, einer Rolle oder einer Zerstäubungsvorrichtung erfolgt.

9. Zusammensetzung zur Behandlung einer Oberfläche mit einem Flächeninhalt von wenigstens 1 m² gegen Kontaminierung durch Mikroorganismen, die zur Durchführung des Verfahrens gemäß einem der Ansprüche 1 bis 8 geeignet ist, wobei die Zusammensetzung aus einer wässrigen Lösung eines hydrophilen polymeren Materials besteht, wobei die Zusammensetzung Teilchen aus pyrogener Kieselsäure in einer Endkonzentration von zu 50 g/l bis 250 g/l umfasst und wenigstens ein Bakterizid oder Konservierungsmittel umfasst.

## Claims

1. A method for reducing the contamination properties by micro-organisms of a surface made of a mineral material, said surface having an area of at least 0.1 m², said method including the following steps:
a) application, onto the surface to be treated, of a layer of a solution or of an aqueous suspension of an hydrophilic polymeric material, such that the said hydrophilic polymeric material adheres on the surface to be treated by the creation of noncovalent bonds between the mineral material of the surface to be treated and the layer of the polymeric material, comprising an hardening agent consisting of silica particles in dispersion in the aqueous liquid medium, said aqueous suspension having a content of silica particles ranging from 50 g.l⁻¹ to 250 g.l⁻¹:
b) drying the surface processed at the step a), for obtaining said surface covered with a layer of said hydrophilic polymeric material, said layer having a surface hardness above 100 N.mm⁻², as determined by a microindentation method, such that an indentor of pyramidal form with top angle of 136° controlled by a microscope penetrates in material under a load of 25 mN in 20 seconds at a temperature of 22 ° C and a water content of 45 %, so that the indentor does not penetrate with more of the quarter thickness of the studied layer.

2. A method according to claim 1, **characterized in that** the mineral material is mineral glass.

3. A method according to claim 1, **characterized in that** the mineral material is selected among ceramic, porcelain, cement or concrete

4. A method according to claim 1, **characterized in that** the mineral material is a metallic material, such as steel or aluminium.

5. A method according to anyone of claims 1 to 4, **characterized in that** the surface of the mineral material consists in the surface of a soil, of a culinary work top, of a table, of a bed, of a reactor, or of a tubing.

6. A method according to anyone of claims 1 to 5, **characterized in that**, in step a), the solution or the aqueous suspension includes the hydrophilic polymeric material at a concentration comprised between 0.5 % and 5 % in weight, preferably between 1 % and 3 % in weight, based on the total weight of the solution or of the aqueous suspension.

7. A method according to claim 6, **characterized in that** the hydrophilic polymeric material is selected among the celluloses and their derivatives, the polyacrylamides and their copolymers, the polyvinylpyrrolidone (PVP) and its copolymers, the vinyl acetate copolymers and vinyl alcohol copolymers, the glycol polyethylenes, the glycol polypropylenes, the hydrophilic polyacrylates, the hydrophilic polymethacrylates, the polyosides and the chitosans.

8. A method according to anyone of claims 1 to 6, **characterized in that** in step a), the application of the hydrophilic polymeric material is conducted by quenching of the surface to be treated, or by application of the material onto the surface using a brush, a roll or a spraying device.

9. A composition for the treatment of a surface having an area of at least 1 m² against contamination by micro-organisms, suitable for implementing the method according to any one of claims 1 to 8, said composition consisting of an aqueous solution of an hydrophilic polymeric material, said composition including fumed silica particles with a final content ranging from 50 g.l⁻¹ to 250 g.l⁻¹, and including at least one bactericidal agent or a preservative agent.
